# EUROPÄISCHE PATENTSCHRIFT

(11) **EP 1 167 359 B1**
(45) Veröffentlichungstag und Bekanntmachung des Hinweises auf die Patenterteilung: **28.04.2004**
(21) Anmeldenummer: 01115113.1
(22) Anmeldetag: 21.06.2001
(51) Int. Cl.: C07D 235/18, C07D 235/20, A61K 7/42

(54) **Verfahren zur Herstellung von 2-Arylbenzimidazolsulfonsäuren**
Process for the preparation of 2-arylbenzimidazole sulphonic acids
Procédé pour la préparation d'acides 2-arylbenzimidazole sulphoniques

(30) Priorität: 23.06.2000 DE 10030664
(43) Veröffentlichungstag der Anmeldung: 02.01.2002
(73) Patentinhaber: MERCK PATENT GmbH, 64293 Darmstadt (DE)
(72) Erfinder: Heywang, Ulrich, Dr., 64289 Darmstadt (DE); Schwarz, Michael, Dr., 64331 Weiterstadt (DE); Pflücker, Frank, Dr., 64297 Darmstadt (DE)

(56) Entgegenhaltungen:
- EP-A- 0 669 323
- WO-A-93/15061
- HOUBEN-WEYL: "Methoden der Organischen Chemie, 4.Auflage, Band 9" 1955 , THIEME VERLAG , STUTTGART XP002172443 * Seite 450 - Seite 455 *
- CHEMICAL ABSTRACTS, vol. 114, no. 5, 4. Februar 1991 (1991-02-04) Columbus, Ohio, US; abstract no. 42654, KUZUETSOV, YU. V. ET AL: "Electrophilic substitution reaction of alkyl-, aryl-, and arylalkyl 5(6)-hydroxybenzimidazole derivatives" XP002172654 & IZV. AKAD. NAUK SSSR, SER. KHIM. (1990), (8), 1888-92 ,

## Beschreibung

Gegenstand der vorliegenden Erfindung sind ein Herstellverfahren für 2-Arylbenzimidazolsulfonsäuren, deren Verwendung als UV-Filter, sowie kosmetische Zubereitungen, die nach dem erfindungsgemäßen Verfahren hergestellte Verbindungen enthalten.

Eine mehr oder minder stark ausgeprägte Sonnenbräune der Haut gilt in der modernen Gesellschaft als attraktiv und als Ausdruck von Dynamik und Sportlichkeit. Neben dieser erwünschten Wirkung der Sonne auf die Haut treten eine Reihe von unerwünschten Nebenwirkungen auf, wie Sonnenbrand oder vorzeitige Hautalterung und Faltenbildung. Inzwischen sind eine Anzahl von leistungsfähigen UV-Filtern entwickelt worden, die in Form von Cremes, Lotionen oder Gelen auf die Haut aufgetragen auch bei stärkerer Sonneneinwirkung die Entwicklung von Sonnenbrand wirksam verzögern können. Der in der pharmazeutischen oder kosmetischen Zubereitung enthaltene UV-Filter bildet auf der Oberfläche der Haut einen Film bzw. eine Schicht aus und dringt nicht mit weiteren in der Zubereitung enthaltenen pflegenden Substanzen in tiefere Hautschichten vor. Bekannte UV-Filter bzw. Sonnenschutzmittel wirken also nur in der Weise, dass sie bestimmte Bereiche des Sonnenlichts absorbieren und somit diese Strahlung nicht in tiefere Schichten der Haut vordringen kann. Bekanntlich wird der gefährlichste Teil der Sonnenstrahlung von den ultravioletten Strahlen mit einer Wellenlänge von weniger als 400 nm gebildet. Die untere Grenze der ultravioletten Strahlen, welche die Erdoberfläche erreichen, ist durch die Absorption in der Ozonschicht auf ca. 280 nm beschränkt. Die heute in der Kosmetik üblichen Sonnenschutzfilter absorbieren in einem Wellenlängenbereich von 280 bis 400 nm. Dieser Bereich umfasst UV-B-Strahlen mit einer Wellenlänge zwischen 280 und 320 nm, die bei der Bildung eines Sonnenerythems eine entscheidende Rollen spielen, wie auch UV-A-Strahlen, mit einer Wellenlänge zwischen 320 und 400 nm, welche die Haut bräunen aber auch altern lassen, die Auslösung einer erythematösen Reaktion begünstigen oder diese Reaktion bei bestimmten Menschen vergrößern oder sogar phototoxische oder photoallergische und irritative Reaktionen auslösen können.

Aufgabe pflegender Kosmetik ist es, nach Möglichkeit den Eindruck einer jugendlichen Haut zu erhalten. Prinzipiell stehen verschiedene Wege offen, um diesen Weg zu erreichen. So können bereits vorhandene Schädigungen der Haut, wie unregelmäßige Pigmentierung oder Faltenbildung, durch abdeckende Puder oder Cremes ausgeglichen werden. Ein anderer Ansatzpunkt ist, die Haut vor Umwelteinflüssen zu schützen, die zu einer dauerhaften Schädigung und damit Alterung der Haut führen. Die Idee ist also, vorbeugend einzugreifen und dadurch den Alterungsprozess hinauszuzögern. Ein Beispiel sind hierfür die bereits erwähnten UV-Filter, welche durch Absorption bestimmter Wellenlängenbereiche eine Schädigung der Haut vermeiden oder zumindest vermindern. Entsprechend der Lage ihrer Absorptionsmaxima werden UV-Absorber für kosmetische und dermatologische Zubereitungen in UV-A- und UV-B-Absorber eingeteilt, wobei UV-A-Absorber üblicherweise auch im UV-B-Bereich absorbieren und daher alternativ auch als Breitbandabsorber oder -filter bezeichnet werden.

Für die Formulierung von entscheidender Bedeutung ist die Löslichkeit der Filtersubstanzen in Öl- und Wasserphasen, da es insbesondere zur Einstellung eines hohen Schutzfaktors erforderlich ist, Filter in alle Phasen der Formulierung einzuarbeiten. Zu den öllöslichen UV-B-Filtern zählen Methoxyzimtsäureisooctylester, Methoxyzimtsäureisoamylester, und Methylbenzylidencampher. Unter den wasserlöslichen UV- Filtern sind insbesondere die Salze der 2-Phenylbenzimidazol-5-sulfonsäure zu nennen, deren Verwendung als UV-Strahlenfilter bereits im Deutschen Reichspatent Nr. 676 103 beschrieben wird.

Zur Herstellung von Arylbenzimidazolsulfonsäuren sind verschiedene Verfahren bekannt. Eine Übersicht über die Herstellung von 2-substituierten Benzimidazolen findet man z. B. in Chemical Reviews Vol. 74, No. 3, 1974 p 279 ff. z. B. erfolgt die Herstellung nach V.G. Sayapin et al., KhGC [Chemistry of Heterocyclic Compounds] 6, 1970, 630-632 in einer Zweistufenreaktion, bei der von 1,2-Phenylendiamin und dem Bisulfitaddukt des Benzaldehyds bzw. von Phenylendiamin und Benzoesäure in Gegenwart von Polyphosphorsäure ausgehend 2-Phenylbenzimidazol hergestellt wird, welches dann mit Chlorsulfonsäure umgesetzt wird.

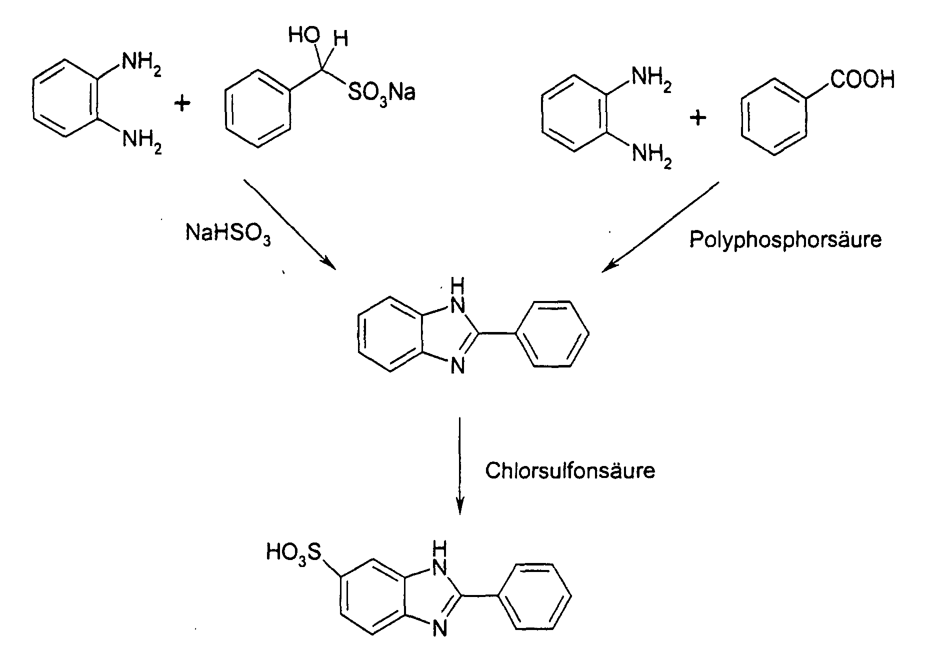

Dieses Verfahren hat jedoch große Nachteile:
- Es ist ein Zweistufenverfahren und somit aufwendig und teuer.
- Bei dem ersten Herstellungsverfahren von Phenylbenzimidazol
   - muss Natriumhydrogensulfit im großen Überschuss eingesetzt werden, so dass im Verlauf der Aufarbeitung große Mengen Schwefeldioxid frei werden.
   - kann als Nebenprodukt 1-Benzyl-2-phenylbenzimidazol entstehen, welches sich nur schlecht abtrennen lässt.
   - entsteht als Nebenprodukt elmentarer Schwefel in kolloidaler, fein verteilter Form der sich bis ins Endprodukt verschleppen kann.
- Bei dem zweiten Verfahren zur Herstellung von Phenylbenzimidazol aus Benzoesäure gelangt Phosphorsäure ins Abwasser, dies ist wegen der Gewässereutrophierung unerwünscht.

In der internationalen Anmeldung WO 93/15061 ist ein Verfahren beschrieben, bei dem durch Umsetzung von o-Phenylendiamin mit Arylcarbonsäuren in 96%iger Schwefelsäure als Lösungsmittel in einem einstufigen Verfahren direkt monosulfonierte Produkte erhalten werden.

Der Einsatz von Chlorsulfonsäure zur Herstellung von Bisbenzimidazoloylsulfonsäuren in einem einstufigen Verfahren ist in der Europäischen Patentanmeldung EP-A-669 323 beschrieben. Dabei wird durch die Chlorsulfonsäure eine zweifache Sulfonierung der Benzimidazoleinheiten erreicht. Mit dem Einsatz von Chlorsulfonsäure sind jedoch verschiedene Probleme verbunden:
- während der Umsetzung erfolgt eine Gasentwicklung (HCI), so dass eine Druckregelung erforderlich ist,
- Abfangen und Entsorgen des aggressiven Gases (HCI) ist erforderlich,
- für die anfallende chloridhaltige Schwefelsäure sind extrem korrosionsbeständige Apparaturen erforderlich,
- Recycling der aus der Verwendung von Chlorsulfonsäure resultierenden chloridhaltigen Schwefelsäure ist nur schwer möglich.

Daher bestand Bedarf nach einem alternativen Verfahren zur Herstellung von Arylbenzimidazolsulfonsäuren, insbesondere von mindestens zweifach sulfonierten Arylbenzimidazolsulfonsäuren, bei dem die oben genannten Probleme nicht auftreten.

Jetzt wurde überraschend gefunden, dass die Herstellung der besagten Arylbenzimidazolsulfonsäuren auch unter Verwendung von Oleum anstelle von Chlorsulfonsäure möglich ist, wobei die oben genannten Probleme vermieden werden.

Ein erster Gegenstand der vorliegenden Erfindung ist daher ein Verfahren zur Herstellung von 2-Arylbenzimidazolsulfonsäuren der Formel I, 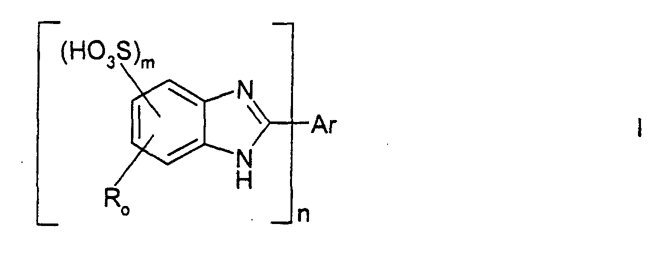 worin Ar ein substituierter oder unsubstituierter Phenyl- oder Naphthylrest und R ein C₁₋₈-Alkyl- oder C₁₋₈-Alkoxy-Rest ist, n 1,2, 3 oder 4, m 2 oder 3 und o 0, 1 oder 2 bedeutet, welches dadurch gekennzeichnet ist, dass ein o-Phenylendiamin gemäß Formel II 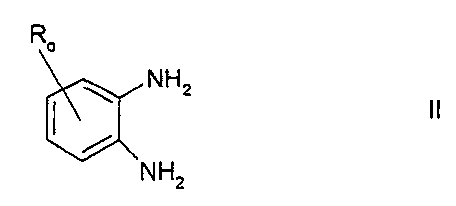 in Gegenwart von Oleum mit einer Verbindung gemäß Formel III 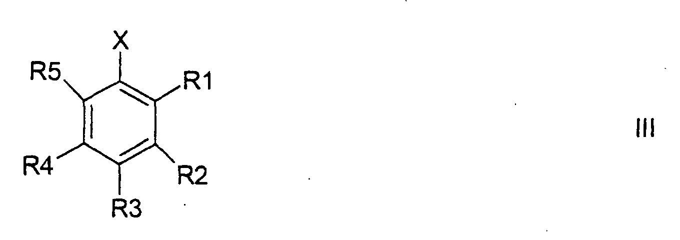 umgesetzt wird, wobei R1, R2, R3, R4 und R5 jeweils unabhängig voneinander stehen für einen Rest aus der Gruppe der H-, C₁₋₈-Alkyl-, C₁₋₈-Alkoxy-, Hydroxy-, Nitro-, F-, Cl-, Br-, I-, COOH-, COOR-, COCI-, COBr- oder CN-Reste und X ausgewählt ist aus den Resten -COOH, -COOR, -COCl, -COBr oder -CN, wobei es sich bei R1 und R2 gemeinsam oder R2 und R3 gemeinsam auch um eine ankondensierte, ggf. substituierte Phenyleneinheit handeln kann und R ein C₁₋₂₀-Alkylrest ist und wobei die Umsetzüng bei Temperatüren zwischen 20°C und 200°C erfolgt und pro mol o-Phenylendiamine nach Formel II mindestens 4 mol Schwefeltrioxid eingesetzt werden.

Dabei weist der Einsatz von Oleum (Schwefeltrioxid) zur Aktivierung der Schwefelsäure im Vergleich zum Einsatz von Chlorsulfonsäure verschiedene Vorteile auf:
- eine Gasentwicklung (HCI) während der Umsetzung erfolgt nicht, so dass eine Druckregelung nicht erforderlich ist,
- dementsprechend ist auch das Abfangen und Entsorgen des aggressiven Gases (HCI) nicht erforderlich,
- während für die anfallende chloridhaltige Schwefelsäure bei Verwendung von Chlorsulfonsäure extrem korrosionsbeständige Apparaturen erforderlich sind, kann mit Oleum in weniger aufwendigen Geräten gearbeitet werden,
- nach der Hydrolyse des Oleums liegt Schwefelsäure vor, die recycliert werden kann, während das Recycling der aus der Verwendung von Chlorsulfonsäure resultierenden chloridhaltigen Schwefelsäure nur schwer möglich ist.

Unter 2-Arylbenzimidazolsulfonsäuren im Sinne der vorliegenden Erfindung werden dabei grundsätzlich auch die Salze dieser Säuren verstanden, wobei Salze vorzugsweise die Alkalimetallsalze, insbesondere die Natrium- oder Kaliumsalze, bzw. die Ammoniumsalze, insbesondere die Triethanolammoniumsalze der entsprechenden Sulfonsäuren sind. Die Herstellung dieser Salze aus den Säuren erfolgt üblicherweise durch Umsetzung der Sulfonsäure mit einer Base, wie Natronlauge, Kalilauge oder einem Amin, bereitet dem Fachmann keinerlei Schwierigkeiten und kann ausdrücklich Bestandteil des erfindungsgemäßen Verfahrens sein.

Dabei werden erfindungsgemäß Arylbenzimidazolsulfonsäuren hergestellt die an jedem Benzimidazolring 2- oder 3-fach, vorzugsweise 2-fäch sulfoniert sind; d.h. bei einem erfindungsgemäß bevorzugten Verfahren werden Verbindungen der Formel I hergestellt, wobei m gleich 2 ist. Erfindungsgemäß besonders bevorzugt ist beispielsweise ein Verfahren zur Herstellung von 2-Phenylbenzimidazol-4,6-disulfonsäure (Formel Ia; hier ist die bei Benzimidazolsulfonsäuren üblicherweise vorliegende Betainform angedeutet), 1,4-Bis-(2-benzimidazoloyl)-benzol-4,4',6,6'-tetrasulfonsäure (Formel Ib: entspricht 2,2'-(1,4-Phenylen)bis1H-benzimidazol-4,6-disulfonsäure) oder 1,3,5-Tris-(2-benzimidazoloyl)-benzol-4,4',4",6,6',6"-tetrasulfonsäure (Formel Ic; entspricht 2,2',2"-(1,3,5-Phenylen)bis1H-benzimidazol-4,6-disulfonsäure). Nach dem erfindungsgemäßen Verfahren lässt sich jedoch bei geeigneter Wahl der Reaktionsbedingengen auch die 2,2'-(1,4-Phenylen)bis-1H-benzimidazol-5-sulfonsäure (Formel Id; entspricht 1,4-Bis-(2-benzimidazoloyl)-benzol-5,5-disulfonsäure) herstellen.

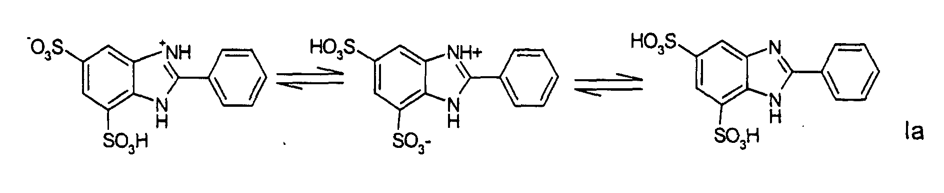

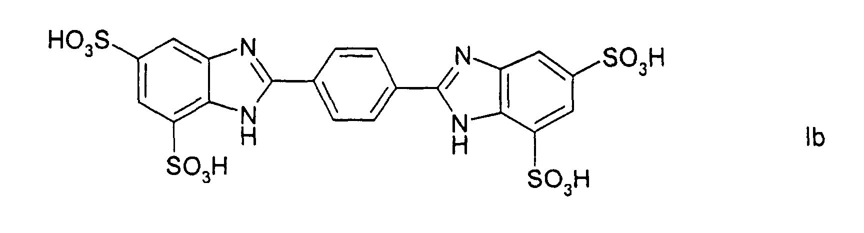

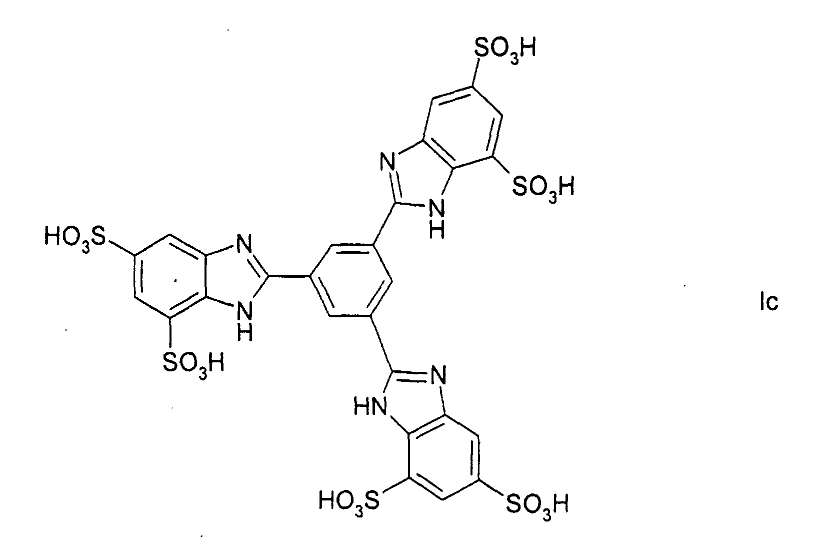

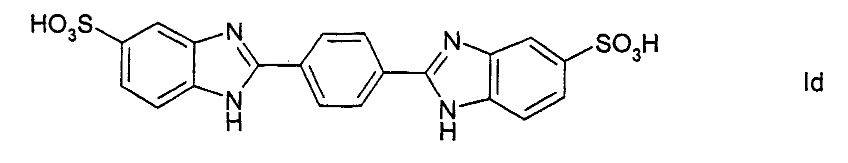

Dabei lässt sich die Sulfonierung über die Reaktionsbedingungen steuern. Insbesondere ist es zur Erreichung der zweifachen Sulfonierung an jedem Benzimidzolring erforderlich, dass Oleum in einer Menge und Konzentration eingesetzt wird, die die zweifache Sulfonierung erlaubt. Bereits für den Ringschluss zum Benzimidazol aus dem o-Phenylendiamin mit einer Carbonsäure werden pro mol o-Phenylendiamin zwei mol Schwefeltrioxid verbraucht. Für jede Sulfonierung wird wiederum 1 Schwefeltrioxid verbraucht, so dass für eine Disulfonierung mindestens 4 mol Schwefeltrioxid pro mol o-Phenylendiamin vorhanden sein müssen. Entsprechend viel Oleum muss folglich eingesetzt werden.

Dabei erfolgt die Umsetzung bei Temperaturen zwischen 20°C und 200°C, vorzugsweise zwischen 160°C und 190°C. Üblicherweise wird die Reaktionstemperatur für 2 bis 8 Stunden aufrecht erhalten; bei Reaktionszeiten von weniger als 2 Stunden werden noch Monosulfonierungsprodukte beobachtet, die sich vom bevorzugten, disulfonierten Produkt nur schwer abtrennen lassen.

Insbesondere bevorzugt ist es erfindungsgemäß, wenn es sich bei der Gruppe X in Verbindung III um eine Gruppierung -COOH oder eine Gruppierung -COOR, wobei R für einen C₁₋₂₀-Alkylrest, vorzugsweise einen C₁₋₈-Alkylrest und insbesondere bevorzugt einen Methyl- oder Ethylrest steht, handelt und die Verbindung vorzugsweise aus der Gruppe Benzoesäure, Salicylsäure, Phthalsäure, Terephthalsäure, Isophthalsäure, 1,2,3-Benzoltricarbonsäure, 1,2,4-Benzoltricarbonsäure, 1,3,5-Benzoltricarbonsäure, 1,2,4,5-Benzoltetracarbonsäure, 1-Naphthalincarbonsäure, 2-Naphthalincarbonsäure, 3-Naphthalincarbonsäure ausgewählt ist. Ganz besonders bevorzugt werden in dem erfindungsgemäßen Verfahren Benzoesäure, Terephthalsäure, Phthalsäure oder 1,3,5-Benzoltricarbonsäure als Verbindung III eingesetzt.

Die Durchführung des erfindungsgemäßen Verfahrens an sich ist einfach und bereitet dem Fachmann keinerlei Schwierigkeiten. Im folgenden ist eine bevorzugte Ausführungsform der Reaktionsdurchführung angegeben, die als Beispiel dienen kann, die möglichen Varianten der Reaktionsführung jedoch nicht einschränken soll:
Man legt die Schwefelsäure, vorzugsweise in Form einer 50-100%igen Lösung, insbesondere als konzentrierte ca. 96%ige Lösung vor und trägt das ortho-Phenylendiamin bzw. dessen Derivat darin ein. Anschließend erfolgt die Aktivierung mit Oleum (z.B. einer 65%ige Lösung von Schwefeltrioxid in Schwefelsäure), wobei die Temperatur vorzugsweise unter 150°C gehalten wird. Dabei wird die Menge an Schwefeltrioxid so gewählt, dass das gesamte, während der Reaktion freiwerdende Wasser abgefangen werden kann. D.h. zur Herstellung einer Benzimidazoldisulfonsäure werden pro mol o-Phenylendiamin mindestens 4 mol Schwefeltrioxid eingesetzt.
Die Zugabe des zweiten Reaktionspartners (Arylcarbonsäure bzw - derivat) erfolgt aus Sicherheitsgründen vorzugsweise erst nach Abkühlung auf eine Temperatur unterhalb 100°C, da sich die Temperatur der Reaktionsmischung durch die Zugabe gegebenenfalls weiter erhöht. Zweckmäßig erfolgt dabei der Einsatz von Arylcarbonsäure in dem mol-Verhältnis zu dem Phenylendiamin in dem im Zielmolekül die Aryleinheit zu den Benzimidazoleinheiten steht. D.h. zur Herstellung von Monobenzimidazol-benzol-derivaten wird eine Arylmonocarbonsäure oder ein Arylmonocarbonsäurederivat im Verhältnis 1:1 mit o-Phenylendiamin eingesetzt, zur Herstellung von Bisbenzimidazol-benzol-derivaten eine Aryldicarbonsäure oder ein Aryldicarbonsäurderivat im Verhältnis 1:2 mit o-Phenylendiamin und z.B. zur Herstellung von Trisbenzimidazol-benzolderivaten eine Aryltricarbonsäure oder ein Aryltricarbonsäurderivat im Verhältnis 1:3 mit o-Phenylendiamin.
Anschließend wird das Reaktionsgemisch langsam auf Temperaturen zwischen 150 und 250 °C, vorzugsweise zwischen 160 und 200 °C erwärmt und 2 bis 8 Stunden bei dieser Temperatur, gegebenenfalls unter Rühren, gehalten. Anschließend kühlt man die Reaktionsmischung, vorzugsweise auf Temperaturen unter 10°C ab und fügt zur Hydrolyse Wasser hinzu.

Nach kurzem Rühren, vorzugsweise 20 Minuten bis 2 Stunden, werden die festen Bestandteile abgetrennt, vorzugsweise mit warmen Wasser gewaschen und getrocknet.
Zur Reinigung des getrockneten Rohproduktes wird dieses vorzugsweise in Natronlauge gelöst und die so erhaltene Lösung, vorzugsweise mit Aktivkohle, gereinigt. Aus der farblosen Lösung wird das Endprodukt mittels einer Säure, vorzugsweise einer Mineralsäure, beispielsweise Schwefelsäure, ausgefällt.

Aufgrund ihrer Absorptionmaxima im UV- Bereich eignen sich die erfindungsgemäß hergestellten 2-Arylbenzimidazolsulfonsäuren als UV-Filtersubstanzen. Dabei zeigt sich, dass die erfindungsgemäß hergestellten 2-Phenylbenzimidazolsulfonsäuren im UV-B-Bereich absorbieren, während die erfindungsgemäß hergestellten Bis- und Trisbenzimidazoyl-Verbindungen Breitbandfilter sind, die sowohl im UV-A- als auch im UV-B-Bereich absorbieren.

Aufgrund dieser Verwendungsmöglichkeit eignen sich die nach dem erfindungsgemäßen Verfahren hergestellten Substanzen hervorragend zum Einsatz in kosmetischen Zubereitungen.

Die schützende Wirkung dieser Zubereitungen gegen UV-Strahlung kann verbessert werden, wenn die Formulierung neben dem erfindungsgemäß hergestellten einen oder mehrere weitere UV-Filter enthält.

Prinzipiell kommen alle UV-Filter für eine Kombination in Frage. Besonders bevorzugt sind solche UV-Filter, deren physiologische Unbedenklichkeit bereits nachgewiesen ist. Sowohl für UVA wie auch UVB-Filter gibt es viele aus der Fachliteratur bekannte und bewährte Substanzen, z.B.
- Benzylidenkampferderivate wie 3-(4'-Methylbenzyliden)-dl-kampfer (z.B. Eusolex® 6300), 3-Benzylidenkampfer (z.B. Mexoryl® SD), Polymere von N-{(2 und 4)-[(2-oxoborn-3-yliden)methyl]benzyl}-acrylamid (z.B. Mexoryl® SW), N,N,N-Trimethyl-4-(2-oxoborn-3-ylidenmethyl)anilinium methylsulfat (z.B. Mexoryl® SK) oder α-(2-Oxoborn-3-yliden)toluol-4-sulfonsäure (z.B. Mexoryl® SL),
- Benzoyl- oder Dibenzoylmethane wie 1-(4-tert-Butylphenyl)-3-(4-methoxyphenyl)propan-1,3-dion (z.B. Eusolex® 9020) oder 4-Isopropyldibenzoylmethan,
- Benzophenone wie 2-Hydroxy-4-methoxybenzophenon (z.B. Eusolex® 4360) oder 2-Hydroxy-4-methoxybenzophenon-5-sulfonsäure und ihr Natriumsalz (z.B. Uvinul® MS-40),
- Methoxyzimtsäureester wie Methoxyzimtsäureoctylester (z.B. Eusolex® 2292), 4-Methoxyzimtsäureisopentylester, z.B. als Gemisch der Isomere (z.B. Neo Heliopan® E 1000),
- Salicylatderivate wie 2-Ethylhexylsalicylat (z.B. Eusolex® OS), 4-Isopropylbenzylsalicylat (z.B. Megasol®) oder 3,3,5-Trimethylcyclohexylsalicylat (z.B. Eusolex® HMS),
- 4-Aminobenzoesäure und Derivate wie 4-Aminobenzoesäure, 4-(Dimethylamino)benzoesäure-2-ethylhexylester (z.B. Eusolex® 6007), ethoxylierter 4-Aminobenzoesäureethylester (z.B. Uvinul® P25),
- weitere Benzimidazolderivate wie 2-Phenylbenzimidazol-5-sulfonsäure sowie ihre Kalium-, Natrium-, Lithium-, Ammonium- und Triethanolaminsalze (z.B. Eusolex® 232), 2,2'-(1,4-Phenylen)bis-(1Hbenzimidazol-4,6-disulfonsäure, Mononatriumsalz) (CAS-Nr. 180 898-37-7) und 2,2'-(1,4-Phenylen)bis-(1H-benzimidazol-5-sulfonsäure) sowie ihre Kalium-, Natrium- und Triethanolaminsalze
- und weitere Substanzen wie 2-Cyano-3,3-diphenylacrylsäure-2-ethylhexylester (z.B. Eusolex® OCR), 3,3'-(1,4-Phenylendimethylen)-bis-(7,7-dimethyl-2-oxobicyclo-[2.2.1]hept-1-ylmethansulfonsäure sowie ihre Salze (z.B. Mexoryl® SX), 2,4,6-Trianilino-(p-carbo-2'-ethylhexyl-1'-oxi)-1,3,5-triazin ( z.B. Uvinul® T 150), 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole®), 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB), α-(Trimethylsilyl)-ω-[trimethylsilyl)oxy]poly[oxy(dimethyl [und ca. 6% methyl[2-[p-[2,2-bis(ethoxycarbonyl]vinyl]phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] (n ≈ 60) (CAS-Nr. 207 574-74-1), 2,2'-Methylen-bis-(6-(2H-benzotriazol-2-yl)-4-(1,1,3,3-tetramethylbutyl)-phenol) (CAS-Nr. 103 597-45-1), und 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6).. Die in der Liste aufgeführten Verbindungen sind nur als Beispiele aufzufassen. Selbstverständlich können auch andere UV-Filter verwendet werden. Diese organischen UV-Filter werden wie auch die erfindungsgemäß hergestellten 2-Arylbenzimidazolsulfonsäuren in der Regel in einer Menge von 0,5 bis 20 Gew.-%, vorzugsweise in einer Menge von 1 - 15 Gew.-% und insbesondere bevorzugt in Mengen von 2 bis 8 Gew.-% je Einzelsubstanz in kosmetische Formulierungen eingearbeitet. Insgesamt enthalten die kosmetischen Zubereitungen üblicherweise bis zu 40 Gew.-%, vorzugsweise 5 bis 25 Gew.-% solcher organischer UV-Filter.

Als anorganische UV-Filter sind solche aus der Gruppe der Titandioxide wie z.B. gecoatetes Titandioxid (z.B. Eusolex® T-2000, Eusolex® T-AQUA), Zinkoxide (z.B. Sachtotec®), Eisenoxide oder auch Ceroxide denkbar. Diese anorganischen UV-Filter werden in der Regel in einer Menge von 0,5 bis 20 Gewichtsprozent, vorzugsweise 2 - 10 %, in kosmetische Formulierungen eingearbeitet.

Werden verschiedene anorganische oder organische UV-Filter eingesetzt, so können diese in nahezu beliebigen Verhältnissen zueinander verwendet werden. Üblicherweise liegen die Verhältnisse der einzelnen Substanzen zueinander im Bereich 1:10-10:1, vorzugsweise im Bereich 1:5-5:1 und insbesondere bevorzugt im Bereich 1:2 - 2:1. Werden UV-A- neben UV-B-Filtern eingesetzt, so ist es für die meisten Anwendungen von Vorteil und daher erfindungsgemäß bevorzugt, wenn der Anteil an UV-B-Filtern überwiegt und das Verhältnis von UV-A-Filtem : UV-B-Filtern im Bereich 1:1 bis 1:3 liegt.

Neben den 2-Arylbenzimidazolsulfonsäuren sind als bevorzugte Verbindungen mit UV-filternden Eigenschaften für die kosmetischen Zubereitungen 3-(4'-Methylbenzyliden)-dl-kampfer, 1-(4-tert-Butylphenyl)-3-(4-methoxyphenyl)propan-1,3-dion, 4-Isopropyldibenzoylmethan, 2-Hydroxy-4-methoxybenzophenon, Methoxyzimtsäureoctylester, 3,3,5-Trimethylcyclohexylsalicylat, 4-(Dimethylamino)benzoesäure-2-ethylhexylester, 2-Cyano-3,3-diphenylacrylsäure-2-ethylhexylester und gecoatetes Titandioxid zu nennen.

Die schützende Wirkung gegen oxidativen Stress bzw. gegen die Einwirkung von Radikalen kann weiter verbessert werden, wenn die Formulierung ein oder mehrere Antioxidantien enthält.

Es gibt viele aus der Fachliteratur bekannte und bewährte Substanzen, die verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin,' Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Diaurylthiodipropionat, Distearylthiodipropionat, Thiodipropiosäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutin bzw. Salze der Schwefelsäureester von Rutin und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercetin, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen kosmetischen Formulierungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex® AP), natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004),

Die Formulierungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂) in den erfindungsgemäßen kosmetischen Formulierungen enthalten, insbesondere bevorzugt Vitamin-A-Palmitat, Vitamin C, DL-α-Tocopherol, Tocopherol-E-Acetat, Nicotinsäure, Pantothensäure und Biotin.

Die erfindungsgemäß hergestellten Verbindungen können in der üblichen Weise in kosmetische Formulierungen eingearbeitet werden. Geeignet sind Formulierungen für eine äußerliche Anwendung, beispielsweise als Creme, Lotion, Gel, oder als Lösung, die auf die Haut aufgesprüht werden kann. Dabei ist es bevorzugt, wenn die Zubereitung mindestens eine Öl- und mindestens eine Wasser-Phase enthält, wobei die erfindungsgemäß hergestellte 2-Arylbenzimidazolsulfonsäure in mindestens einer wässrigen Phase enthalten ist.

Als Anwendungsform der kosmetischen oder pharmazeutischen Formulierungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Formulierung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnußöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typisch kosmetische Anwendungsformen sind auch Lippenstifte, Lippenpflegestifte, Mascara, Eyeliner, Lidschatten, Rouge, Puder-, Emulsionsund Wachs-Make up sowie Sonnenschutz-, Prä-Sun- und After-Sun-Präparate.

Alle Verbindungen oder Komponenten, die in den kosmetischen Formulierungen verwendet werden können, sind entweder bekannt und käuflich erwerbbar oder können nach bekannten Verfahren synthetisiert werden.

Die kosmetische Zubereitung eignet sich besonders zum Schutz menschlicher Haut vor den schädigenden Einflüssen der UV-Anteile im Sonnenlicht, daneben bieten sie auch Schutz gegen Alterungsprozesse der Haut sowie vor oxidativem Stress, d.h. gegen Schädigungen durch Radikale, wie sie z.B. durch Sonneneinstrahlung, Wärme oder andere Einflüsse erzeugt werden. Dabei liegt sie in verschiedenen, für diese Anwendung üblicherweise verwendeten Darreichungsformen vor. So kann sie insbesondere als Lotion oder Emulsion, wie als Creme oder Milch (O/W, W/O, O/W/O, W/O/W), in Form öligalkoholischer, ölig-wässriger oder wässrig-alkoholischer Gele bzw. Lösungen, als feste Stifte vorliegen oder als Aerosol konfektioniert sein.

Die Formulierung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und außer der oder den erfindungsgemäß hergestellten 2-Arylbenzimidazolsulfonsäuren und vorzugsweise weiteren UV-Filtem beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen Ölen und Wachsen, Lanolin, Fettsäureestem, insbesondere Triglyceriden von Fettsäuren, oder ölig-alkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die kosmetische Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Die kosmetische Formulierung kann auch zum Schutz der Haare gegen photochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Formulierung vor oder nach dem Shamponieren, vor oder nach dem Färben oder Entfärben bzw. vor oder nach der Dauerwelle aufgetragen wird. Es kann auch eine Formulierung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die kosmetische Formulierung kann außer der oder den erfindungsgemäßen 2-Arylbenzimidazolsulfonsäure und weiteren UV-Filtern verschiedene, in diesem Mitteltyp verwendete Adjuvantien enthalten, wie grenzflächenaktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Die kosmetischen Zubereitungen können mit Hilfe von Techniken hergestellt werden, die dem Fachmann wohl bekannt sind.

Zum Schutz der Haut und/oder natürlicher oder sensibilisierter Haare vor Sonnenstrahlen wird auf die Haut oder die Haare eine kosmetische Zubereitung, enthaltend eine oder mehrere erfindungsgemäß hergestellte Verbindungen aufgetragen. Als sensibilisierte Haare werden dabei Haare verstanden, welche einer chemischen Behandlung, wie einer Dauerwellenbehandlung, einem Färbeoder Entfärbeprozeß unterzogen worden sind.

Ferner wirken die erfindungsgemäß hergestellten 2-Arylbenzimidazolsulfonsäuren auch stabilisierend auf die Formulierung. Bei der Verwendung in entsprechenden Produkten bleiben diese daher auch länger stabil und verändern ihr Aussehen nicht. Insbesondere bleibt auch bei längerdauernder Anwendung bzw. längerer Lagerung die Wirksamkeit der Inhaltsstoffe, z.B. Vitamine, erhalten. Dies ist besonders vorteilhaft bei der Zusammensetzungen zum Schutz der Haut gegen die Einwirkung von UV-Strahlen, da diese Kosmetika besonders hohen Belastungen durch die UV-Strahlung ausgesetzt sind.

Eine bekannte und leistungsfähige Klasse von Lichtschutzfiltersubstanzen wird von den Dibenzoylmethanderivaten gebildet. Nachteilig ist jedoch, daß diese Substanzen sehr leicht durch UV-Licht zersetzt werden und damit ihre schützenden Eigenschaften verlorengehen. Als Beispiel für einen auf dem Markt erhältlichen Lichtschutzfilter aus dieser Verbindungsklasse sei das 4-(tert.-Butyl)-4'-methoxydibenzoylmethan angeführt, welches die in Formel IV gezeigte Struktur aufweist.

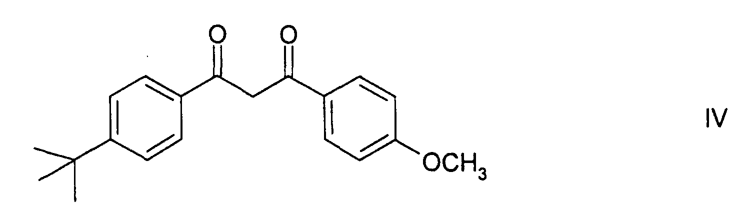

Überraschend hat sich nun gezeigt, dass erfindungsgemäß hergestellte 2-Arylbenzimidazolsulfonsäuren eine sehr gute Stabilisierungswirkung für die Dibenzoylmethane, insbesondere 4-(tert.-buthyl)-4-methoxibenzoylmethan, aufweisen. Indem Gemische dieser Verbindungen in Kosmetika eingearbeitet werden, ist es nun möglich, Lichtschutzmittel unter Verwendung von Dibenzoylmethanen herzustellen, die auch bei längerer Sonneneinwirkung, beispielsweise während eines mehrstündigen Sonnenbades, keine oder nur eine geringe Verringerung der Schutzwirkung gegen UV-Strahlen aufweisen.

In den folgenden Beispielen wird die vorliegende Erfindung näher erläutert, ohne den Umfang der Erfindung zu beschränken. Dabei werden in den Beispielrezepturen die folgenden Handelsnamen verwendet:
**Antaron® V-220** wird von der Fa. GAF, Frechen, DE, vertrieben,
**Carbomer Ultrez-10** wird von der Fa. Goodrich, Neuss, DE, angeboten,
**Dehymuls® E** ist eine Mischung aus Dicocolypentaerytritylcitrat, Sorbitolsesquioleat, Bienenwachs und Aluminiumstearat und wird von der Fa. Cogni, Roermond, NL, vertrieben,
**Eusolex® 2292, Eusolex®232, Eusolex® 6300** und **Eusolex® HMS** sind UV-Filter, die von der Merck KGaA, Darmstadt, DE, vertrieben werden,
**Luvitol® EHO** wird von der Fa. BASF AG, Ludwigshafen, DE, vertrieben,
**Pemulen® TR-1** und **Pemulen®TR-2** sind Acrylat/ Alkylacrylatpolymere, die von der Fa. Goodrich, Neuss, DE, vertrieben werden,
**Performa® V825** ist ein synthetisches Wachs, das von der Fa. New Phase, NJ08554, US, vertrieben wird,
**Oxynex® K** ist eine Mischung aus PEG-8, Tocophenol, Ascorbylpalmitat, Ascorbinsäure und Zitronensäure und wird von der Merck KGaA, Darmstadt, DE, vertrieben.

Die in den Rezepturbeispielen 3 bis 9 angegebenen Sulfonsäuren (2-Phenylbenzimidazol-4,6-disulfonsäure, 1,4-Bis-(2-benzimidazoloyl)-benzol-4,4',6,6'-tetrasulfonsäure, 1,4-Bis-(2-benzimidazoloyl)-benzol-5,5'-disulfonsäure) wurden nach dem erfindungsgemäßen Verfahren hergestellt.

### Beispiel 1: Herstellung von 2-Phenylbenzimidazol-4,6-disulfonsäure

108 Teile o-Phenylendiamin werden in 500 Teile H₂SO₄ (> 96%) eingetragen und anschließend werden 800 Teile Oleum (65%ig) zugetropft, wobei die Temperatur bei maximal 120°C gehalten wird. Nach 15 min wird auf 70°C abgekühlt und es werden 120 Teile Benzoesäure zugegeben. Danach wird für 2 h auf 180°C erhitzt. Anschließend wird mit 2500 Teilen Wasser langsam hydrolysiert, wobei die Temperatur unter 10°C gehalten wird. Der Niederschlag (Kristalle) wird abgesaugt, in 8 Teilen Wasser suspendiert und mit Natronlauge (32 %ig) bei pH = 7 gelöst. Es wird mit Aktivkohle gerührt, bis die Lösung farblos ist, und anschließend mit 96 %iger H₂SO₄ bei pH = 1-2 gefällt. Es werden 300 Teile 2-Phenylbenzimidazol-4,6-disulfonsäure erhalten. Die Verbindung besitzt ein Absorptionsmaximum im UV-B-Bereich bei λₘₐₓ = 308 nm.

Analog werden hergestellt:
2-(4'-Methoxyphenyl)benzimidazol-4,6-disulfonsäure
2-(3'-Methoxyphenyl)benzimidazol-4,6-disulfonsäure
2-(4'-Ethoxyphenyl)benzimidazol-4,6-disulfonsäure
2-(3'-Ethoxyphenyl)benzimidazol-4,6-disulfonsäure
2-(3', 5'-Dimethoxyphenyl)benzimidazol-4,6-disulfonsäure
2-(3',5'-Diethoxyphenyl)benzimidazol-4,6-disulfonsäure
2-(3',4'-Diethoxyphenyl)benzimidazol-4,6-disulfonsäure
2-(2'-Naphthyl)-benzimidazol-4,6-disulfonsäure.

### Beispiel 2: Herstellung von 1,4-Bis-(2-benzimidazoloyl)-benzol-4,4',6,6'tetrasulfonsäure

108 Teile o-Phenylendiamin werden in 500 Teile H₂SO₄ (> 96%) eingetragen und anschließend werden 800 Teile Oleum (65%ig) zugetropft, wobei die Temperatur bei maximal 120°C gehalten wird. Nach 15 min wird auf 70°C abgekühlt und es werden 83 Teile Terephthalsäure zugegeben. anschließend wird für 2 h auf 180°C erhitzt. Anschließend wird mit 2500 Teilen Wasser langsam hydrolysiert, wobei die Temperatur unter 10°C gehalten wird. Der Niederschlag (Kristalle) wird abgesaugt, in 8 Teilen Wasser suspendiert und mit Natronlauge (32 %ig) bei pH = 7 gelöst. Es wird mit Aktivkohle gerührt, bis die Lösung farblos ist, und anschließend mit 96 %iger H₂SO₄ bei pH = 1-2 gefällt. Es werden 270 Teile 1,4-Bis-(2-benzimidazoloyl)-benzol-4,4',6,6'-tetrasulfonsäure erhalten. Die Verbindung besitzt Absorptionsmaxima bei λₘₐₓ = 208 nm, 257 nm und 335 nm.

Analog werden hergestellt:
1,4-Bis-(2-benzimidazoloyl)-3-methoxy-benzol-4,4',6,6'-tetrasulfonsäure
1,4-Bis-(2-benzimidazoloyl)-3,5-dimethoxy-benzol-4,4',6,6'-tetrasulfonsäure
1,4-Bis-(2-benzimidazoloyl)-3-ethoxy-benzol-4,4',6,6'-tetrasulfonsäure

### Beispiel 3: Sonnenschutzsprav (O/W)

| Phase | Inhaltsstoff | **Gew.-%** |
|---|---|---|
| **A** | Eusolex® 2292 (Art.-Nr. 105382) | 7,50 |
| | Eusolex® HMS (Art.-Nr. 111412) | 7,00 |
| | Steareth-2 | 0,40 |
| | Steareth-10 | 0,80 |
| | Pemulen® TR-2 | 0,18 |
| | Propylen-Glycol Isoceteth-3 Acetat | 5,00 |
| | Performa® V 825 | 0,80 |
| | Dimeticon | 1,00 |
| | Oxynex® K (Art.-Nr. 108324) | 0,10 |
| **B** | 2-Phenylbenzimidazol-4,6-disulfonsäure | 1,00 |
| | Triethanolamin | 0,90 |
| | Propandiol-1,2 | 2,00 |
| | Konservierungsmittel | 0,50 |
| | Wasser demineralisiert | a.d. 100,00 |

### Herstellung:

**Phase B:** Das Wasser wird mit dem Triethanolamin vermischt und anschließend die 2-Phenylbenzimidazol-4,6-disulfonsäure unter Rühren zugegeben. Sobald sich alles gelöst hat, werden die weiteren Bestandteile der Phase B zugegeben und anschließend die Mischung auf 80°C erwärmt.
**Phase A:** Die Bestandteile der Phase A mit Ausnahme von Permulen® TR-2 werden zusammengegeben und auf 80°C erwärmt. Anschließend wird das Permulen® TR-2 unter Rühren zugegeben.
**Herstellung des Sonnenschutzmittels:** Zur Phase A wird unter Rühren langsam die Phase B gegeben. Nach dem Homogenisieren wird unter Rühren abgekühlt.
Als Konservierungsmittel werden verwendet 0,05 % Propyl-4-hydroxybenzoat und 0,15 % Methyl-4-hydroxybenzoat.

### Beispiel 4: Sonnenschutzspray (O/W)

| Phase | Inhaltsstoff | **Gew.-%** |
|---|---|---|
| **A** | Eusolex® 2292 (Art.-Nr. 105382) | 7,50 |
| | Eusolex® HMS (Art.-Nr. 111412) | 7,00 |
| | Steareth-2 | 0,40 |
| | Steareth-10 | 0,80 |
| | Pemulen® TR-2 | 0,18 |
| | Propylen-Glycolisoceteth-3-acetat | 5,00 |
| | Performa® V 825 | 0,80 |
| | Dimeticon | 1,00 |
| | Oxynex® K (Art.-Nr. 108324) | 0,10 |
| **B** | 2-Phenylbenzimidazol-4,6-disulfonsäure | 1,00 |
| | 1,4-Bis-(2-benzimidazoloyl)-benzol-4,4',6,6'-tetrasulfonsäure | 1,00 |
| | Triethanolamin | 0,90 |
| | Propandiol-1,2 | 2,00 |
| | Wasser demineralisiert | a.d. 100,00 |

### Herstellung:

**Phase B:** Das Wasser wird mit dem Triethanolamin vermischt und anschließend die 1,4-Bis-(2-benzimidazoloyl)-benzol-4,4',6,6'-tetrasulfonsäure sowie die 2-Phenylbenzimidazol-4,6-disulfonsäure unter Rühren zugegeben. Sobald sich alles gelöst hat, werden die weiteren Bestandteile der Phase B zugegeben und anschließend die Mischung auf 80°C erwärmt.
**Phase A:** Die Bestandteile der Phase A mit Ausnahme von Permulen® TR-2 werden zusammengegeben und auf 80°C erwärmt. Anschließend wird das Permulen® TR-2 unter Rühren zugegeben.
**Herstellung des Sonnenschutzmittels:** Zur Phase A wird unter Rühren langsam die Phase B gegeben. Nach dem Homogenisieren wird unter Rühren abgekühlt.
Als Konservierungsmittel werden verwendet 0,05 % Propyl-4-hydroxybenzoat und 0,15 % Methyl-4-hydroxybenzoat.

### Beispiel 5: Sonnenschutzgel (wässriq)

| Phase | Inhaltsstoff | **Gew.-%** |
|---|---|---|
| **A** | 1,4-Bis-(2-benzimidazoloyl)-benzol-4,4',6,6'-tetrasulfonsäure | 1,00 |
| | Eusolex® 232 (Art.-Nr. 105372) | 4,00 |
| | Natriumhydroxidlösung | 6,00 |
| | Glycerin | 3,00 |
| | Propandiol-1,2 | 2,00 |
| | Konservierungsmittel | q.s. |
| | Wasser demineralisiert | a.d. 100,00 |
| **B** | Carbomer Ultrez-10 | 0,70 |
| | Wasser, demineralisiert | 60,00 |
| **C** | Natriumhydroxidlösung (10 %) | 1,50 |
| | Wasser, demineralisiert | 4,00 |

### Herstellung:

Carbomer Ultrez-10 wird im Wasser der Phase B vollständig dispergiert. Anschließend wird langsam die Phase C zugegeben und homogenisiert. Für die Phase A wird zunächst das Wasser zur Natriumhydroxidlösung gegeben und dann das Eusolex® 232 zugegeben und unter Rühren vollständig gelöst. Nach Erhalt einer klaren Lösung werden die weiteren Bestandteile der Phase A zugegeben. Anschließend wird Phase A portionsweise zum Gemisch der Phasen B und C gegeben, wobei nach jeder Zugabe homogenisiert wird.
Als Konservierungsmittel wird verwendet:
0,20 % Methyl-4-hydroxybenzoat

### Beispiel 6: Sonnenschutzgel (O/W)

| Phase | Inhaltsstoff | **Gew.-%** |
|---|---|---|
| **A** | Eusolex® 6300 (Art.-Nr. 5385) | 0,75 |
| | Luvitol® EHO | 10,00 |
| | Dimeticon | 2,00 |
| | Sheabutter | 5,00 |
| | Antaron® V-220 | 2,00 |
| | Oxynex® K | 1,00 |
| **B** | 1,4-Bis-(2-benzimidazoloyl)-benzol-5,5'-disulfonsäure | 1,00 |
| | Eusolex® 232 (Art.-Nr. 105372) | 0,75 |
| | Tris(hydroxymethyl)-aminomethan | 0,33 |
| | Konservierungsmittel | q.s. |
| | Wasser demineralisiert | 20,00 |
| **C** | Tris(hydroxymethyl)-aminomethan | 1,20 |
| | Wasser, demineralisiert | 10,00 |
| **D** | Pemulen® TR-1 | 0,60 |
| | Wasser, demineralisiert | ad 100,00 |

### Herstellung:

Das Pemulen® TR-1 wird im Wasser der Phase D gelöst. Das Tris(hydroxymethyl)aminomethan wird im Wasser der Phase C gelöst und die Lösung zur Phase D gegeben. Das Tris(hydroxymethyl)aminomethan wird im Wasser der Phase B gelöst und dann unter Rühren das Eusolex® 232 zugegeben. Nach Erhalt einer klaren Lösung weden die weiteren Bestandteile der Phase B zugegeben und dann die Phase B zum Gemisch der Phasen C und D gegeben und homogenisiert. Die Bestandteile der Phase A werden vermengt und erwärmt. Dann wird Phase A unter Homogenisieren zum Gemisch der übrigen Phasen gegeben.
Als Konservierungsmittel werden verwendet 0,05 % Propyl-4-hydroxybenzoat und 0,15 % Methyl-4-hydroxybenzoat.

### Beispiel 7: Sonnenschutzgel (O/W)

| **Phase** | **Inhaltsstoff** | **Gew.-%** |
|---|---|---|
| **A** | Eusolex® 6300 (Art.-Nr. 5385) | 0,75 |
| | Luvitol® EHO | 10,00 |
| | Dimeticon | 2,00 |
| | Sheabutter | 5,00 |
| | Antaron® V-220 | 2,00 |
| | Oxynex® K liquid (Art.-Nr. 8324) | 1,00 |
| **B** | 2-Phenylbenzimidazol-4,6-disulfonsäure | 1,00 |
| | 1,4-Bis-(2-benzimidazoloyl)-benzol-4,4',6,6'-tetrasulfonsäure | 0,75 |
| | Tris(hydroxymethyl)-aminomethan | 0,33 |
| | Konservierungsmittel | q.s. |
| | Wasser demineralisiert | 20,00 |
| **C** | Tris(hydroxymethyl)-aminomethan | 1,20 |
| | Wasser, demineralisiert | 10,00 |
| **D** | Pemulen® TR-1 | 0,60 |
| | Wasser, demineralisiert | ad 100,00 |

### Herstellung:

Das Pemulen® TR-1 wird im Wasser der Phase D gelöst. Das Tris(hydroxymethyl)aminomethan wird im Wasser der Phase C gelöst und die Lösung zur Phase D gegeben. Das Tris(hydroxymethyl)aminomethan wird im Wasser der Phase B gelöst und dann unter Rühren die 2-Phenylbenzimidazol-4,6-disulfonsäure und die 1,4-Bis-(2-benzimidazoloyl)-benzol-4,4',6,6'tetrasulfonsäure zugegeben. Nach Erhalt einer klaren Lösung werden die weiteren Bestandteile der Phase B zugegeben und dann die Phase B zum Gemisch der Phasen C und D gegeben und homogenisiert. Die Bestandteile der Phase A werden vermengt und erwärmt. Dann wird Phase A unter Homogenisieren zum Gemisch der übrigen Phasen gegeben.
Als Konservierungsmittel wird verwendet 0,05 % Propyl-4-hydroxybenzoat und 0,15 % Methyl-4-hydroxybenzoat.

### Beispiel 8: Sonnenschutzlotion (W/O) mit UVA/B-Schutz

| Phase | Inhaltsstoff | **Gew.-%** |
|---|---|---|
| **A** | Eusolex®2292 (Art.-Nr. 105382) | 3,00 |
| | Eusolex®4360 (Art.-Nr. 1.05376) | 2,00 |
| | Dehymuls® E | 6,00 |
| | gehärtetes Castoröl | 1,00 |
| | Bienenwachs | 2,00 |
| | Oleylerucat | 6,00 |
| | Decycloeat | 6,00 |
| | Dimeticon | 1,00 |
| | Dicaprylether | 5,00 |
| **B** | Glycerin (87 %) | 5,00 |
| | 1,4-Bis-(2-benzimidazoloyl)-benzol-4,4',6,6'-tetrasulfonsäure | 3,00 |
| | Magnesiumsulfate Heptahydrat | 1,00 |
| | Konservierungsmittel | q.s. |
| | Wasser demineralisiert | a.d. 100,00 |

### Herstellung:

Die Bestandteile der Phasen A und B werden jeweils vermengt. Phase A wird auf 75°C und Phase B getrennt auf 80°C erwärmt. Phase B wird unter Homogenisieren zu Phase A gegeben. Anschließend wird unter Rühren abgekühlt.

Als Konservierungsmittel werden verwendet:
0,05 % Propyl-4-hydroxybenzoate
0,15 % Methyl-4-hydroxybenzoate.

### Beispiel 9: Sonnenschutzlotion (W/O) mit UVA/B-Schutz

| Phase | Inhaltsstoff | **Gew.-%** |
|---|---|---|
| **A** | Eusolex®2292 (Art.-Nr. 105382) | 3,00 |
| | Eusolex®4360 (Art.-Nr. 1.05376) | 2,00 |
| | Dehymuls® E | 6,00 |
| | gehärtetes Castoröl | 1,00 |
| | Bienenwachs | 2,00 |
| | Oleylerucat | 6,00 |
| | Decycloeat | 6,00 |
| | Dimeticon | 1,00 |
| | Dicaprylether | 5,00 |
| **B** | 2-Phenylbenzimidazol-4,6-disulfonsäure | 2,00 |
| | 1,4-Bis-(2-benzimidazoloyl)-benzol-5,5'-disulfonsäure | 2,00 |
| | Glycerin (87 %) | 5,00 |
| | Magnesiumsulfate Heptahydrat | 1,00 |
| | Konservierungsmittel | q.s. |
| | Wasser demineralisiert | a.d. 100,00 |

### Herstellung:

Die Bestandteile der Phasen A und B werden jeweils vermengt. Phase A wird auf 75°C erwärmt und getrennt davon Phase B auf 80°C. Phase B wird unter Homogenisierung zu Phase A gegeben. Anschließend wird unter Rühren abgekühlt.

Als Konservierungsmittel werden verwendet:
0,05 % Propyl-4-hydroxybenzoat
0,15 % Methyl-4-hydroxybenzoat.

## Patentansprüche

1. Verfahren zur Herstellung von 2-Arylbenzimidazolsulfonsäuren der Formel I, 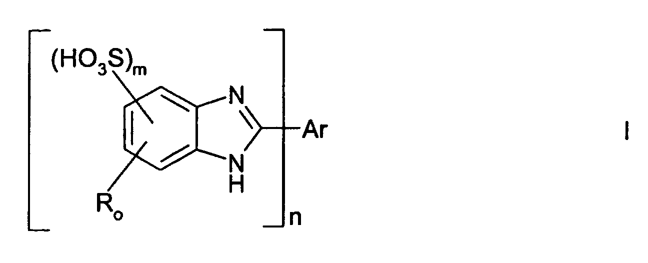 worin
Ar ein substituierter oder unsubstituierter Phenyl- oder Naphthylrest und
R ein C₁₋₈-Alkyl- oder C₁₋₈-Alkoxy-Rest ist
n 1,2, 3 oder 4,
m 2 oder 3 und
o 0, 1 oder 2 bedeutet,
**dadurch gekennzeichnet, dass** ein o-Phenylendiamin gemäß Formel II 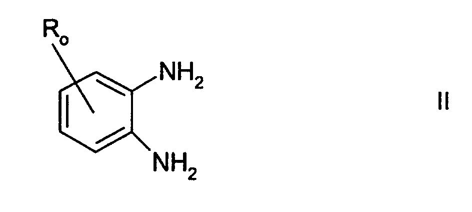 in Gegenwart von Oleum mit einer Verbindung gemäß Formel III 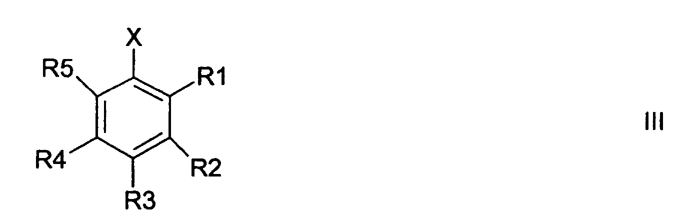 umgesetzt wird, wobei R1, R2, R3, R4 und R5 jeweils unabhängig voneinander stehen für einen Rest aus der Gruppe der H-, C₁₋₈-Alkyl-, C₁₋₈-Alkoxy-, Hydroxy-, Nitro-, F-, Cl-, Br-, I-, COOH-, COOR-, COCI-, COBr- oder CN-Reste und X ausgewählt ist aus den Resten -COOH, -COOR, -COCI, -COBr oder -CN, wobei es sich bei R1 und R2 gemeinsam oder R2 und R3 gemeinsam auch um eine ankondensierte, ggf. substituierte Phenyleneinheit handeln kann und R ein C₁₋₂₀-Alkylrest ist,
und wobei die Umsetzung bei Temperaturen zwischen 20°C und 200°C erfolgt und pro mol o-Phenylendiamin nach Formel II mindestens 4 mol Schwefeltrioxid eingesetzt werden.

2. Verfahren nach Anspruch 1, **dadurch gekennzeichnet, dass** die Umsetzung bei Temperaturen zwischen 160°C und 190°C erfolgt.

3. Verfahren nach einem der Ansprüche 1 bis 2, **dadurch gekennzeichnet, dass** es sich bei der Gruppe X in Verbindung III um eine Gruppierung -COOH oder eine Gruppierung -COOR, wobei R für einen C₁₋₂₀-Alkylrest, vorzugsweise einen C₁₋₈-Alkylrest und insbesondere bevorzugt einen Methyl- oder Ethylrest steht, handelt und die Verbindung vorzugsweise aus der Gruppe Benzoesäure, Salicylsäure, Phthalsäure, Terephthalsäure, Isophthalsäure, 1,2,3-Benzoltricarbonsäure, 1,2,4-Benzoltricarbonsäure, 1,3,5-Benzoltricarbonsäure, 1,2,4,5-Benzoltetracarbonsäure, 1-Naphthalincarbonsäure, 2-Naphthalincarbonsäure, 3-Naphthalincarbonsäure ausgewählt ist, wobei es sich insbesondere bevorzugt um Benzosäure, Terephthalsäure, Phthalsäure oder 1,3,5-Benzoltricarbonsäure handelt.

## Claims

1. Process for the preparation of 2-arylbenzimidazolesulfonic acids of the formula I 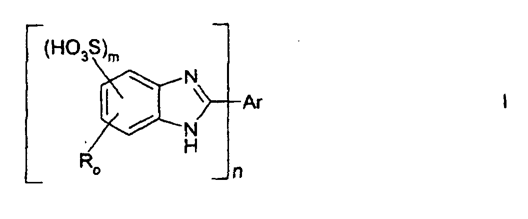 in which
Ar is a substituted or unsubstituted phenyl or naphthyl radical and
R is a C₁₋₈-alkyl or C₁₋₈-alkoxy radical
n is 1, 2, 3 or 4,
m is 2 or 3 and
o is 0, 1 or 2,
**characterized in that** a o-phenylenediamine according to formula II 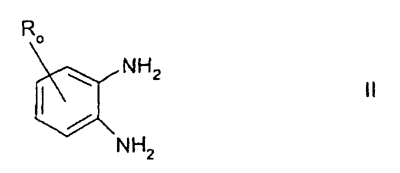 is reacted in the presence of oleum with a compound according to formula III 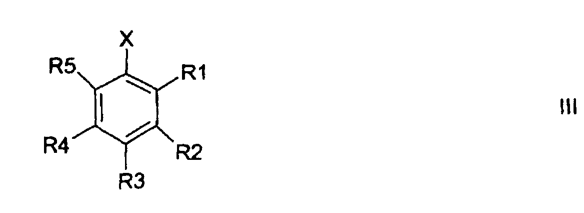 where R1, R2, R3, R4 and R5, in each case independently of one another, are a radical from the group of H, C₁₋₈-alkyl, C₁₋₈-alkoxy, hydroxyl, nitro, F, Cl, Br, I, COOH, COOR, COCl, COBr or CN radicals, and X is chosen from the radicals -COOH, - COOR, -COCl, -COBr, or -CN, where R1 and R2 together or R2 and R3 together may also be a fused-on, optionally substituted phenylene unit, and R is a C₁₋₂₀-alkyl radical and where the reaction takes place at temperatures between 20°C and 200°C and at least 4 mol of sulphur trioxide are used per mol of o-phenylenediamine according to formula II.

2. Process according to Claim 1, **characterized in that** the reaction takes place at temperatures between 160°C and 190°C.

3. Process according to one of Claims 1 to 2, **characterized in that** the group X in compound III is a -COOH group or a -COOR group, where R is a C₁₋₂₀-alkyl radical, preferably a C₁₋₈-alkyl radical and particularly preferably a methyl or ethyl radical, and the compound is preferably chosen from the group consisting of benzoic acid, salicylic acid, phthalic acid, terephthalic acid, isophthalic acid, 1,2,3-benzenetricarboxylic acid, 1,2,4-benzenetricarboxylic acid, 1,3,5-benzenetricarboxylic acid, 1,2,4,5-benzenetetracarboxylic acid, 1-naphthalenecarboxylic acid, 2-naphthalenecarboxylic acid, 3-naphthalenecarboxylic acid, particular preference being given to benzoic acid, terephthalic acid, phthalic acid or 1,3,5-benzenetricarboxylic acid.

## Revendications

1. Procédé pour la préparation des acides 2-arylbenzimidazole sulfonique de formule I 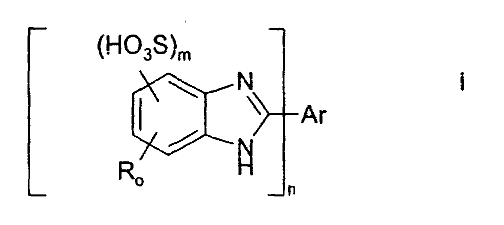 où
Ar est un reste phényle ou naphtyle substitué ou non et
R est un reste alkyle en C₁₋₈ ou un reste alcoxy en C₁₋₈,
n est égal à 1, 2, 3 ou 4,
m à 2 ou 3 et
o à 0, 1 ou 2
**caractérisé en ce que** l'on fait réagir une o-phénylènediamine conforme à la formule II 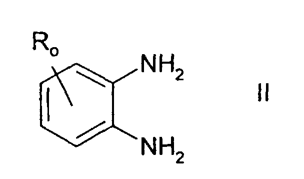 en présence d'oléum avec un composé conforme à la formule III 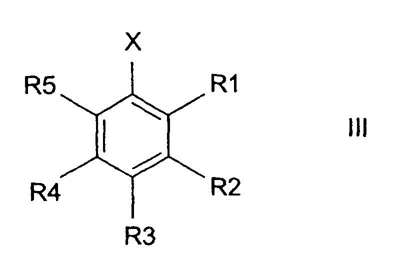 où R1, R2, R3, R4 et R5 représentent, indépendamment les uns des autres, un reste provenant du groupe constitué par les restes H, alkyles en C₁₋₈, alcoxy en C₁₋₈, hydroxy, nitro, F, CI, Br, I, COOH, COOR, COCl, COBr ou CN et X est choisi parmi les restes -COOH, -COOR, -COCl, -COBr ou -CN, R1 et R2 ensemble ou R2 et R3 ensemble pouvant être également un motif phénylène condensé, éventuellement substitué et R étant un reste alkyle en C₁₋₂₀, la réaction s'effectuant à des températures comprises entre 20°C et 200°C et en utilisant par mole d'o-phénylènediamine selon la formule II au moins 4 moles d'anhydride sulfurique.

2. Procédé selon la revendication 1, **caractérisé en ce que** la réaction s'effectue à des températures comprises entre 160°C et 190°C.

3. Procédé selon l'une des revendications 1 et 2, **caractérisé en ce que** le groupe X dans le composé III est un groupement -COOH ou un groupement -COOR, où R représente un reste alkyle en C₁₋₂₀, de préférence un reste alkyle en C₁₋₈ et plus particulièrement, de préférence, un reste méthyle ou éthyle et le composé est choisi, de préférence, dans le groupe des acides benzoïque, salicylique, phtalique, téréphtalique, isophtalique, 1,2,3-benzène-tricarboxylique, 1,2,4-benzène-tricarboxylique, 1,3,5-benzène-tricarboxylique, 1,2,4,5-benzène-tétracarboxylique, 1-naphtalène-carboxylique, 2-naptalène-carboxylique, 3-naphtalène-carboxylique, les acides benzoïque, téréphtalique, phtalique ou 1,3,5-benzène-tricarboxylique étant plus particulièrement préférés.
